# EUROPEAN PATENT SPECIFICATION

(11) **EP 0 328 932 B1**
(45) Date of publication and mention of the grant of the patent: **14.07.1993**
(21) Application number: 89101663.6
(22) Date of filing: 01.02.1989
(51) Int. Cl.: B01L 3/14

(54) **Body fluid sample collection tube assembly**
Röhrchen zur Aufnahme einer Körperflüssigkeitsprobe
Tube collecteur d'échantillon de liquide corporel

(30) Priority: 19.02.1988 US 158192
(43) Date of publication of application: 23.08.1989
(73) Proprietor: Becton, Dickinson and Company, Franklin Lakes, New Jersey 07417-1880 (US)
(72) Inventor: Dufresne, Christopher M., Tappan New York 10983 (US); Plucinski, Andrzej J., Norwood New Jersey 07648 (US); Nalezny, Casmir P., Paramus New Jersey 07652 (US)
(74) Representative: Selting, Günther, Dipl.-Ing.

(56) References cited:
- EP-A- 0 295 416
- DE-A- 2 603 392
- DE-B- 1 757 030
- US-A- 2 493 380
- US-A- 4 625 884

## Description

This invention relates to containers for receiving body fluid samples, and for containing those samples for subsequent examination to determine the presence or absence of disease in the samples. Generally speaking, such containers are in tube form and they may or may not be evacuated, depending upon the particular sample being taken. As will be understood by practitioners-in-the-art, evacuated tubes are used in great numbers for taking blood samples with the tubes frequently containing reagents for reacting with the blood samples for determining the presence or absence of disease.

The tubes may also be non-evacuated tubes for taking samples for one reason or another. Of course urine samples may also be taken in both evacuated and non-evacuated tubes. While non-evacuated tubes are utilized in great numbers, it is preferred to use evacuated tubes for many specific applications for maintaining a seal of the tube prior to use and for facilitating the entry of the sample into the evacuated tube for subsequent testing of the sample.

For evacuated tubes, in particular, it is important to maintain the vacuum over a period of time in order to provide appropriate storage life for those tubes prior to their being used. That is, it is important for the vacuum level to be maintained for a period of time prior to the time when a technician or a nurse uses the tube for receiving a blood sample, for example.

Many developments have been made in the past in order to provide plastic tubes for evacuated tube applications. However, plastic tubes have not been developed to the extent where they will maintain an appropriate vacuum for a period of time long enough to be satisfactory for a shelf-life which is appropriate under the circumstances in which such tubes are used. Moreover, various plastics have a tendency, in certain applications, to react adversely or interfere with blood chemistries, subsequently, during the actual testing of the sample in a lab. In some instances, the plastic may interfere with reactants contained in the tube for the purpose of reacting with a blood sample, for example, in order to provide an appropriate test result during examination.

Therefore, it is appropriate and conventional for tubes to be comprised of glass for use in evacuated tubes because glass maintains the vacuum for a much longer or indefinite period of time, and glass does not react adversely in most cases with any blood chemistry applications utilizing such tubes.

The difficulty, on the other hand, with the use of glass tubes is breakage. With the advent of the highly contagious AIDS virus in many people, it has become extremely important to avoid contamination of technicians, nurses and doctors by blood samples obtained by them from diseased patients.

As will be understood, glass tubes break and/or they may be cracked by being struck, inadvertently, against some object during the course of the taking of a sample or the course of the sample being delivered from a patient to the laboratory for subsequent testing. It will be understood, further, that such breakage and/or cracking may result in leakage of a diseased blood sample, for example, over the hands of the technician or the person taking the sample or the laboratory technician who is in the course of examining the sample for the presence of disease.

If that technician happens to have an open wound, the possibility of acquiring the AIDS virus or some other disease such as hepatitis, is substantial. Also, broken glass may cut and contaminate, and the pieces must be handled in order to be disposed of. Accordingly, great pains are being taken in the development of any materials utilized for taking and handling samples which contain diseases of this kind and it is to this situation to which this invention is particularly directed.

DE-A-2 603 392 discloses a test container for blood tests comprising a test tube made from transparent synthetic resin for containing the blood sample. The test tube is inserted into a holding tube of a centrifuge separator for separating the blood components. The open end of the test tube is sealed by an elastomeric stopper and the test tube is held within the holding tube of the centrifuge by an annular distance member made from elastomeric material. The distance member is inserted into the outer holding tube in a position where it does not overlap with the stopper. The axial position with the distance member depends from how far the distance member is pressed into the holding tube.

It is an object of the present invention to provide a composite sample container assembly wherein the inner tube is capable of holding a vacuum for a long time, but which, in case of cracking of the inner tube, avoids leakage of the blood sample.

The composite sample container assembly of the invention comprises the features of claim 1.

The invention utilizes a clear transparent plastic tube configured to receive coaxially therein a clear transparent glass tube. The bottom curved internal surface of the plastic tube may contain ribs integral with the bottom surface of the plastic tube in order to stabilize and maintain the glass tube inserted therein in a co-axial position therewith. Subsequent to insertion of the glass tube within the plastic tube of the assembly of the invention herein, an interlock ring specifically arranged to maintain the coaxial positioning of the outer plastic tube with the inner glass tube is placed adjacent the top edge of the plastic and glass tubes. The interlock ring is specifically configured in order to maintain the integrity of this coaxial positioning and to provide appropriate stability for the assembly of the invention.

In addition, the ring includes an integral annular internal flap which "gives" during insertion of the glass tube into the plastic ring so as to frictionally grip the glass tube and hold it coaxially in place within the plastic tube. Thus, by having the entire outer surface of the glass tube covered by a plastic tube, if the glass tube is broken or cracked, in the kind of accident discussed above, the plastic tube around the glass tube contains the sample therein. Even though the sample may not be utilized for subsequent testing for the presence of disease because of the crack or break, at least the technician may contain the sample and dispose of it prior to any dripping or spilling and subsequent contamination. Also, the plastic tube shields the user from sharp broken edges and contains the shattered pieces of glass.

It will be understood that it makes no difference whether the glass tube is evacuated or not evacuated in accordance with this invention. The presence of this sleeve firmly attached to the outer surface of the glass container holding the sample has the effect of maintaining the general integrity of the container holding a sample so that it may be properly disposed of without any contamination to the user. In addition, the sleeve makes the container stronger over all because of the cushioning characteristics thereof.

As purely illustrative of a plastic sleeve material which may be used over a glass fluid specimen tube, in accordance herewith, one may note that the plastic sleeve or tube is preferably injection molded from a rigid thermoplastic material having high clarity and good impact resistance. Representative materials for this purpose include, for example, polyethylene terephthalate, styrene acrylonitrile, acrylonitrile-butadiene-styrene, polycarbonate, or other thermoplastic materials having the high clarity and good impact resistance required. The interlock ring utilized, in accordance herewith is comprised of a resilient material in order to develop "snap-lock" properties so that the ring may be snapped in place inside and around the top edge of the plastic sleeve, and may again be resiliently deformed for receiving the glass tube therein in order to hold the entire assembly in its desired coaxial position.

As a further feature of the invention, a curable filler or adhesive may be used along a portion of the adjacent opposed surfaces of the glass and plastic tubes particularly near the bottom closed ends thereof in order to maintain the two tubes fixed relative to each other in their desired coaxial position. Moreover, as discussed above, the internal bottom surface of the plastic tube may include integral ribs around the circumferential edge of the bottom internal surface for engaging the outer surface of the glass tube.

With the foregoing and additional objects in view, this invention will not be described in more detail and other objects and advantages thereof will be apparent from the following description, the accompanying drawings, and the appended claims.

As purely illustrative of an arrangement of container assembly which may be used for carrying out this invention, one may note the attached drawings in which a preferred embodiment of such a container is shown utilizing the coaxially arranged glass and plastic container assembly of the invention.

### IN THE DRAWINGS

Fig. 1 is a longitudinal sectional view of a tube-shaped body fluid sample container assembly, illustrating the invention;
Fig. 2 is a side elevational view of the interlock ring of the invention;
Fig. 3 is a cross-sectional view of the interlock ring of the invention;
Fig. 4 is an enlarged sectional view of the annular overhang portion of the interlock ring of the invention; and
Fig. 5 is a bottom plan view of the assembly shown in Fig. 1.

### DETAILED DESCRIPTION OF THE INVENTION

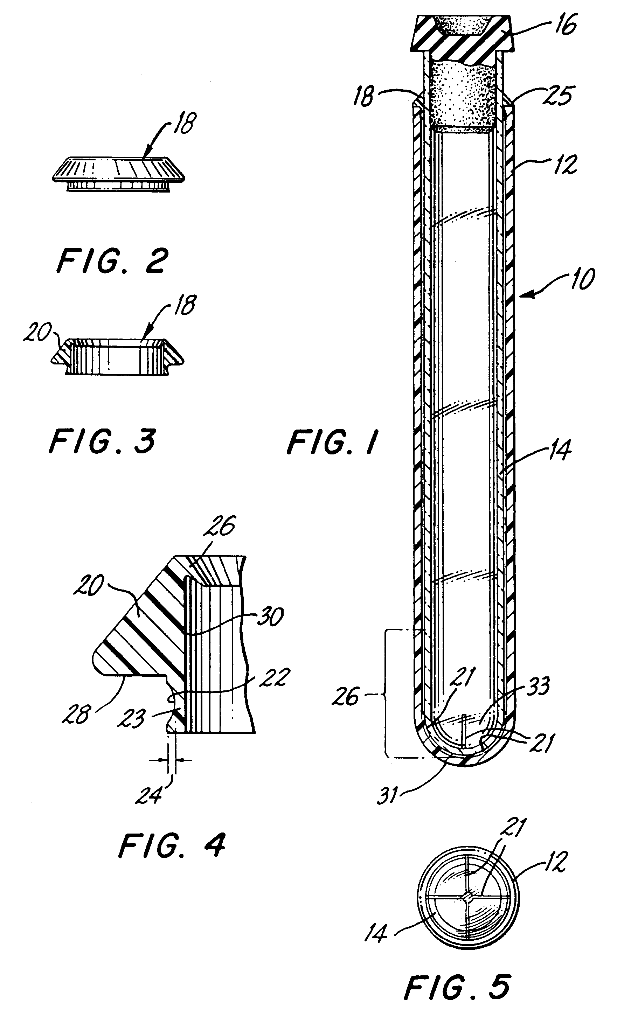

Referring to the drawings in which like reference characters refer to like parts throughout the several views thereof, Fig. 1 shows an assembly generally designated 10 including an outer clear plastic tube 12. Coaxially positioned within tube 12 is a clear glass tube 14. Positioned on the top edge of plastic tube 12 is an interlock ring 18. Received in the top of the glass tube 14 is an elastomer stopper 16 for sealing glass tube 14 and maintaining the vacuum in glass tube 14, if required. At any rate, elastomeric stopper 16 is a conventional stopper of the kind utilized in evacuated blood collection tubes. Also, stopper 16 may be used simply to maintain glass tube 14 sealed for subsequent use. Stopper 16 may be comprised of, for example, natural or synthetic rubber or a combination thereof. Also, stopper 16 may be enclosed in a plastic cap.

Figs. 2 and 3 show an enlarged view of ring 18 which serves for interlocking the glass and the plastic tube in a stabilized coaxial position as shown in Fig. 1. Interlock ring 18 includes an annular portion 20 which is shown in detail in Fig. 4. Annular overlap portion 20 of ring 18 includes an annular depending portion 23 which has a curved insert surface 22 for receiving the top rounded or beaded edge of plastic tube 12. The depth 24 of this curved portion 22 is about 0,127 mm (0.005 inches). Thus, the top edge 25 of tube 12 has a rounded bead, which is conventional in plastic tubes of this kind, and is received within this radial annular insert 22 for holding the interlock ring 18 firmly in place on the top edge 25 of plastic tube 12. Moreover, the overhang surface 28 of annular overhang nortion 20 rests upon the top edge 25 of plastic tube 12.

As shown on the upper internal surface 30 of annular ring 18, an integral flap 26 is positioned. This flap 26 is deformed downwardly and radially outward upon insertion of glass tube 14 through ring 18 in the assembly of the glass tube 14 within the plastic tube 12. This deformation has the effect of having the flap 26 surface frictionally engage and hold glass tube 14 within plastic tube 12 in the desired axial position.

Also positioned in the bottom internal surface of the closed end 31 of plastic tube 12 are a plurality of spaced apart ribs 21 (Fig. 5) integral with the internal surface of the closed end 31 of plastic tube 12. These ribs serve to seat and engage the bottom closed end 33 of glass tube 14. If desired, an adhesive may be used in the general area designated 22 in Fig. 1 between the outer surface of glass tube 14 and the inner surface of plastic tube 12 for adding increased stability and fixing the assembly of the invention so as to maintain the tubes in their desired coaxial position.

Representative adhesives for this purpose include ultraviolet radiation curable adhesives such as IMPRUV R, a product of Loctite Corporation, cyanoacrylate adhesives such as SUPERBONDER R, a product of Loctite Corporation. Other adhesives include heat curable adhesives such as UNISET R, a product of Amicon or an anaerobic adhesive, such as SPEEDBONDER R, a product of Loctite Corporation. A preferred adhesive is the Loctite product, IMPRUV R formulas 362, 365, or 366 which adhesives have indefinite fixing time to facilitate assembly. Formula 363 is particularly preferred because it has high clarity and will not interfere, therefore, with subsequent blood sample observation in the laboratory when the technician is viewing the results of the tests of the sample in the glass tube contained within the plastic tube of the invention.

Thus, as will be apparent from the foregoing, there are provided in accordance with this invention, safety containers for receiving and holding body fluid samples which may or may not contain disease. The arrangement herein of a plastic tube covering the entire glass container is particularly appropriate for evacuated containers since glass tubes may then be used without the danger of cracking and/or breaking the container while it contains a disease containing a body fluid sample. There is a substantial reduction in the possibility of contamination of the user under these circumstances.

It should be borne in mind that many blood sample tubes are subjected to centrifugal forces for separating a blood sample, for example, into its individual components. It is under these conditions, in some circumstances, that the glass tubes are broken flinging glass particles and blood sample within the centrifuge resulting in contamination of the equipment and possibly the environment. With this invention, this situation is obviated.

It will be understood that this invention provides a very useful and inexpensive approach to containing samples in glass containers, whether or not the containers are evacuated. Because of the tremendous concern with the spread of diseases such as hepatitis and AIDS, the arrangement herein is particularly useful for applications of the kind where the potential for spreading the disease is great, and particularly in obtaining blood samples in a series of evacuated tubes for subsequent transfer to a clinical lab for examination for the presence of such a disease.

## Claims

1. A composite sample container assembly (10) for receiving and containing human fluid samples without leakage, comprising
a plastic tube (12) having an open end and a closed end (31), said open end of said plastic tube defining the annular upper edge (25) of said plastic tube;
an inner tube (14) inserted into said open end of said plastic tube (12) so that said plastic tube and said inner tube are positioned coaxially relative to each other, said inner tube having an open end and a closed end (33);
an annular locking ring (18) comprised of a resilient material positioned between said inner tube (14) and said plastic tube (12), and frictionally engaging the outer surface of said inner tube (14) adjacent the said open end of said inner tube; and
an elastomeric stopper (16) sealing the open end of said inner tube (14),
**characterized in that**
said inner tube (14) is a glass tube, and
said annular locking ring (18) is snap-fit onto the upper edge (25) of the open end of the plastic tube (12) and includes an integrated annular resilient flap (26) frictionally engaging the outer surface of the glass tube (14) in a portion surrounding the stopper (16).

2. The assembly of claim 1, wherein said glass tube (14) is evacuated.

3. The assembly of claim 1 or 2, further characterized by
a plurality of integral ribs (21) extending from the internal surface of said closed end (31) of said plastic tube (12);
said ribs (21) being spaced circumferentially around said closed end of said plastic tube; and
the surface of said plurality of ribs (21) engaging the outer surface of said closed end (33) of said glass tube (14) for maintaining the coaxial relationship of said glass and plastic tubes.

4. The assembly of one of claims 1-3, wherein
adhesive is applied to the internal surface of said plastic tube (12) and the external surface of said glass tube (14) adjacent the said closed ends (31,33) of said plastic and glass tubes for maintaining the said coaxial positioning thereof.

5. The assembly of one of claims 1-4, wherein
said annular locking ring (18) includes an annular depending portion (23) having an annular outwardly facing surface, said annular outwardly facing surface having an annular radial insert surface (22) for engaging said annular edge (25) of said open end of said plastic tube (12) in said snap-fit engagement.

## Patentansprüche

1. Zusammengesetzte Probenbehältervorrichtung (10) zum auslaufsicheren Aufnehmen und Halten menschlicher Fluidproben, mit
einem Kunststoffrohr (12) mit einem offenen Ende und einem geschlossenen Ende (31), wobei das offene Ende des Kunststoffrohres den ringförmigen oberen Rand (25) des Kunststoffrohres bildet;
einem Innenrohr (14), das in das offene Ende des Kunststoffrohres (12) derart eingesetzt ist, daß das Kunststoffrohr und das Innenrohr relativ zueinander koaxial angeordnet sind, wobei das Innenrohr ein offenes Ende und ein geschlossenes Ende (33) aufweist;
einem aus elastischem Material bestehenden ringförmigen Verschlußring (18), der zwischen dem Innenrohr (14) und dem Kunststoffrohr (12) angeordnet ist und reibend an der Außenfläche des Innenrohres (14) angrenzend an das offene Ende des Innenrohres angreift; und
einem das offene Ende des Innenrohres (14) abdichtenden Stopfen (16),
**dadurch gekennzeichnet,**
daß das Innenrohr (14) ein Glasrohr ist, und
der ringförmige Verschlußring (18) schnappend auf dem oberen Rand (25) des offenen Endes des Kunststoffrohres (12) angebracht ist und eine integrierte ringförmige elastische Klappe (26) aufweist, die reibend an der Außenfläche des Glasrohres (14) in einem den Stopfen (16) umgebenden Bereich angreift.

2. Vorrichtung nach Anspruch 1, bei der das Glasrohr (14) entleert ist.

3. Vorrichtung nach Anspruch 1 oder 2, ferner gekennzeichnet durch
mehrere sich einstückig von der Innenfläche des geschlossenen Endes (31) des Kunststoffrohres (12) erstreckende Rippen (21);
wobei die Rippen (21) umfangsmäßig um das geschlossene Ende des Kunststoffrohres voneinander beabstandet angeordnet sind; und
die Oberfläche der mehreren Rippen (21) zum Einhalten der koaxialen Beziehung von Glas- und Kunststoffrohr an der Außenfläche des geschlossenen Endes (33) des Glasrohres (14) angreift.

4. Vorrichtung nach einem der Ansprüche 1-3, bei der
Klebemittel auf die Innenfläche des Kunststoffrohres (12) und die Außenfläche des Glasrohres (14) angrenzend an die geschlossenen Enden (31,33) der Kunststoff- und Glasrohre zum Einhalten von deren koaxialer Anordnung aufgebracht ist.

5. Vorrichtung nach einem der Ansprüche 1-4, bei der
der ringförmige Verschlußring (18) einen ringförmigen herabhängenden Bereich (23) aufweist, der eine ringförmige, nach außen weisende Oberfläche aufweist, wobei die ringförmige, nach außen weisende Oberfläche eine ringförmige radiale Einsetzfläche (22) zum Angreifen an dem ringförmigen Rand (25) des offenen Endes des Kunststoffrohres (12) beim schnappenden Eingreifen aufweist.

## Revendications

1. Un ensemble (10) de conteneur composite d'échantillon pour recevoir et contenir sans fuite des échantillons de fluides du corps humain, comprenant
un tube (12) en matière plastique ayant une extrémité ouverte et une extrémité fermée (31) , ladite extrémité ouverte dudit tube en matière plastique définissant le bord supérieur annulaire (25) dudit tube en matière plastique ;
un tube interne (14) inséré dans ladite extrémité ouverte dudit tube (12) en matière plastique afin que ledit tube en matière plastique et ledit tube interne soient disposés coaxialement l'un par rapport à l'autre, ledit tube interne ayant une extrémité ouverte et une extrémité fermée (33);
un anneau de blocage annulaire (18) constitué d'une matière élastique , placé entre ledit tube interne (14) et ledit tube (12) en matière plastique et venant en contact de friction avec la surface externe dudit tube interne (14) près de ladite extrémité ouverte dudit tube interne ; et
un bouchon (16) en matière élastomère étanchéifiant l'extrémité ouverte dudit tube interne (14) , caractérisé en ce que
ledit tube interne (14) est un tube en verre et
ledit anneau de blocage annulaire (18) est à adaptation à pression sur le bord supérieur (25) de l'extrémité ouverte du tube (12) en matière plastique et comprend un volet élastique annulaire intégré (26) venant en contact de friction avec la surface externe du tube en verre (14) dans une partie entourant le bouchon (16).

2. L'ensemble selon la revendication 1 dans lequel le tube en verre (14) est sous vide.

3. L'ensemble selon la revendication 1 ou 2, caractérisé, en outre, par :
une pluralité de nervures solidaires (21) s'étendant de la surface interne de ladite extrémité fermée (31) dudit tube (12) en matière plastique ;
lesdites nervures (21) étant espacées à la périphérie autour de ladite extrémité fermée dudit tube en matière plastique ; et
la surface de ladite pluralité de nervures (21) vient en contact avec la surface externe de ladite extrémité fermée (33) dudit tube en verre (14) pour maintenir la relation coaxiale entre ledit tube en verre et ledit tube en matière plastique .

4. L'ensemble selon l'une des revendications 1 à 3 dans lequel un adhésif est appliqué à la surface interne du tube (12) en matière plastique et à la surface externe du tube en verre (14) près desdites extrémités fermées (31, 33) dudit tube en matière plastique et dudit tube en verre pour maintenir leur positionnement coaxial.

5. L'ensemble selon l'une des revendications 1 à 4 dans lequel ledit anneau de blocage annulaire (18) comprend une partie pendante annulaire (23) ayant une surface annulaire dirigée vers l'extérieur , ladite surface annulaire dirigée vers l'extérieur présentant une surface d'insertion radiale annulaire (22) pour la mise en contact avec ledit bord annulaire (25) de ladite extrémité ouverte dudit tube (12) en matière plastique lors du contact d'adaptation à pression.
